# EUROPEAN PATENT SPECIFICATION

(11) **EP 4 220 122 B1**
(45) Date of publication and mention of the grant of the patent: **23.10.2024**
(21) Application number: 23152413.3
(22) Date of filing: 19.01.2023
(51) Int. Cl.: G01N 1/28, G01N 3/08, G01N 33/24, G01N 1/10

(54) **INDOOR SOIL SAMPLE PREPARATION INSTRUMENT**
INNENGERÄT ZUR VORBEREITUNG VON BODENPRBEN
INSTRUMENT D'INTÉRIEUR DE PRÉPARATION D'ÉCHANTILLONS DE SOL

(30) Priority: 29.01.2022 CN 202210111138
(43) Date of publication of application: 02.08.2023
(73) Proprietor: China Three Gorges Corporation, Wuhan, Hubei 430010 (CN)
(72) Inventor: DAI, Jialin, WUHAN, 430010 (CN); ZHANG, Wei, WUHAN, 430010 (CN); LUO, Lunbo, WUHAN, 430010 (CN); LI, Zhou, WUHAN, 430010 (CN); YU, Guangming, WUHAN, 430010 (CN)
(74) Representative: Lavoix

(56) References cited:
- CN-A- 110 595 886
- KR-B1- 101 334 918
- US-A1- 2018 120 283

## Description

### TECHNICAL FIELD

The present invention relates to the field of geotechnical engineering technology, and specifically to an indoor soil sample preparation instrument.

### BACKGROUND

The quantitative study of the physical and mechanical properties of soft clay requires the preparation of soil samples by indoor consolidation. There are mainly two ideas of common indoor preparation of soil samples for soil tests at present. The first one is to place soft clay slurry in a consolidation box and subsequently pre-consolidate the soft clay slurry on a consolidator. The second one is to use vacuum pre-consolidation, which can accelerate the percolation of pore water in soft clay. After the consolidation is completed, soil samples are obtained from bulk samples in the consolidation box with a sampler to carry out soil tests

US 2018/120283 A1 relates to an expandable jacket used for three-dimensional consolidation and settlement tests to determine consolidation and settlement properties of soils and intermediate geomaterials.

At present, soil samples prepared by using the foregoing methods are usually bulk soil samples, and cannot be directly used for indoor soil tests. Therefore, it is necessary to take samples from bulk soil samples. When an existing sampler is pressed into the bulk soil samples, disturbance to the consolidated soil samples is large, and cutting and separation from surrounding soil body are also difficult.

### SUMMARY

Therefore, a technical problem to be resolved by the present invention is to overcome the problem in the prior art that when an existing sampler is pressed into bulk soil samples, disturbance to consolidated soil samples is large, and cutting and separation from surrounding soil body are also difficult, and provide an indoor soil sample preparation method.

To achieve the above objective, the embodiments of the present invention provide an indoor soil sample preparation instrument as defined in claim 1. The indoor soil sample preparation instrument includes: a consolidation box, with a water filter layer being laid on a bottom thereof, and with a drain provided in the bottom of the consolidation box; and a sampling tube, having a tubular structure, the sampling tube being disposed in the consolidation box, an end of the sampling tube being connected to the water filter layer, a certain angle being formed between the sampling tube and the water filter layer.

Optionally, the consolidation box has a rigid structure.

Optionally, the consolidation box is made of stainless steel or aluminum alloy.

A baffle is disposed at a top of the consolidation box in a circumferential direction.

Optionally, the baffle and the consolidation box are detachably connected to each other.

Optionally, the baffle and the consolidation box are correspondingly provided with a plurality of fixing sheets at a connection location between the baffle and the consolidation box, and a bolt hole for insertion of a bolt is provided in each fixing sheet.

Optionally, a sealing strip is laid at the connection location between the baffle and the consolidation box.

Optionally, a drain valve is installed at the drain.

Optionally, a galvanization process is applied to a tube wall of the sampling tube.

Optionally, the sampling tube is a telescopic tube or is formed by connecting a plurality of tube rods.

The indoor soil sample preparation instrument further includes: a plurality of arc-shaped vertical plates, disposed around a periphery of the sampling tube, the plurality of arc-shaped vertical plates surrounding the sampling tube in a space delimited by the plurality of arc-shaped vertical plates.

Optionally, each arc-shaped vertical plate has a rigid structure.

Optionally, a slot hole is opened in the arc-shaped vertical plate, and a hole pressure gauge is installed in the slot hole.

Optionally, a sealing material is filled between the hole pressure gauge and the slot hole.

The indoor soil sample preparation instrument further includes: a permeable stone, disposed at a bottom of the sampling tube.

A soil pressure detection apparatus is installed on the permeable stone.

Optionally, a through hole is opened in the sampling tube at a position corresponding to the slot hole, a transmission line is provided in the through hole, one end of the transmission line is connected to the soil pressure detection apparatus, and the other end of the transmission line is connected to the hole pressure gauge.

Optionally, a plurality of vertical posts are arranged around the permeable stone.

Optionally, a base ring is installed upon the vertical posts, a plurality of flange boards are disposed at intervals at bottoms of the arc-shaped vertical plates, and the base ring is connected to the flange boards.

Optionally, the water filter layer includes a grit layer and non-woven fabric, the grit layer is laid at the bottom of the consolidation box, the grit layer forms a consolidation drain path, and the non-woven fabric is laid flat on the grit layer.

Compared with the prior art, the technical solution in the present invention has the following advantages:
1. The embodiments of the present invention provide an indoor soil sample preparation instrument. The indoor soil sample preparation instrument includes: a consolidation box, with a water filter layer being laid on a bottom thereof, and with a drain provided in the bottom of the consolidation box; and a sampling tube, having a tubular structure, the sampling tube being disposed in the consolidation box, an end of the sampling tube being connected to the water filter layer, a certain angle being formed between the sampling tube and the water filter layer.
   In such an arrangement, the sampling tube is installed in the consolidation box of the present invention. Compared with pressing of an existing sampler into a large soil sample, a conventional process of pressing a sampling tube into soft clay is not required in the present invention, and sample preparation and sampling are integrated, so that disturbance to soil samples can be avoided, thereby improving sampling quality, and cutting and separation from surrounding soil body are easier.
2. In the embodiments of the present invention, the sampling tube can be kept from corrosion and the tube wall of the sampling tube can be kept in a smooth state by applying the galvanization process to the tube wall of sampling tube, so that disturbance to soil samples can be avoided, thereby improving sampling quality, and cutting and separation from surrounding soil body are easier.
3. In the embodiments of the present invention, the plurality of arc-shaped vertical plates are disposed around the periphery of the sampling tube, and the plurality of arc-shaped vertical plates surround the sampling tube in a space delimited by the plurality of arc-shaped vertical plates, so that the sampling tube can be protected, to keep the sampling tube from bending and damage.
4. In the embodiments of the present invention, the baffle is disposed to adjust the interior space of the instrument and the length of the sampling tube is changed, so that sample preparation of soft clay of different scales can be implemented according to actual test requirements of soft clay in soil tests.
5. In the embodiments of the present invention, the soil pressure detection apparatus and the hole pressure gauge are installed on the sampling tube and the permeable stone, so that the consolidation and preparation of soft clay can be accurately measured and controlled, and the consolidation status of soft clay can be acquired in real time.

### BRIEF DESCRIPTION OF THE DRAWING

To describe the technical solutions in specific embodiments of the present invention or the prior art more clearly, the following briefly introduces the accompanying drawings required for describing the specific embodiments or the prior art. Apparently, the accompanying drawings in the following description show some embodiments of the present invention, and a person of ordinary skill in the art may still derive other drawings from these accompanying drawings without creative efforts.
FIG. 1 is a schematic diagram of an overall structure of an indoor soil sample preparation instrument according to an embodiment of the present invention;
FIG. 2 is an enlarged view of a part A of an indoor soil sample preparation instrument according to an embodiment of the present invention;
FIG. 3 is an enlarged view of a part B of an indoor soil sample preparation instrument according to an embodiment of the present invention;
FIG. 4 is a top view of an overall structure of an indoor soil sample preparation instrument according to an embodiment of the present invention; and
FIG. 5 is an enlarged view of a part C of an indoor soil sample preparation instrument according to an embodiment of the present invention.

### Reference numerals:

1, consolidation box; 2, baffle; 3, fixing sheet; 4, bolt; 5, sealing strip; 6, drain valve; 7, vertical post; 8, permeable stone; 9, flange board; 10, base ring; 11, soil pressure detection apparatus; 12, transmission line; 13, arc-shaped vertical plate; 14, sampling tube; 15, slot hole; 16, hole pressure gauge; 17, sealing material; 18, non-woven fabric; and 19, grit layer.

### DETAILED DESCRIPTION

The following clearly and completely describes the technical solutions of the present invention with reference to the accompanying drawings. Apparently, the described embodiments are merely some rather than all of the embodiments of the present invention. All other embodiments obtained by a person of ordinary skill in the art based on the embodiments of the present invention without creative efforts shall fall within the protection scope of the present invention as defined in the appended claims.

In the description of the present invention, it needs to be understood that orientation or location relationships indicated by terms "center", "up", "down", "left", "right", "vertical", "horizontal", "inside", and "outside" are based on orientation or location relationships shown in the accompanying drawings, and are only used to facilitate description of the present invention and simplify description, but are not used to indicate or imply that the apparatuses or elements must have specific orientations or are constructed and operated by using specific orientations, and therefore, cannot be understood as a limit to the present invention. In addition, the terms "first", "second", and "third" are used only for description, but are not intended to indicate or imply relative importance.

In the description of the present invention, it needs to be noted that unless otherwise expressly specified and defined, "mounted", "connected", and "connection", should be understood in a broad sense, for example, fixedly connected, detachably connected or integrally connected; or mechanically connected or electrically connected; or connected directly or indirectly through an intermediate, or two elements communicated internally, or connected in a wireless manner, or connected in a wired manner. For a person of ordinary skill in the art, specific meanings of the terms in the present invention should be understood according to specific conditions.

In addition, the technical features involved in different embodiments of the present invention described below can be combined with each other as long as they do not constitute a conflict between them.

The quantitative study of the physical and mechanical properties of soft clay requires the preparation of soil samples by indoor consolidation. There are mainly two ideas of common indoor preparation of soil samples for soil tests at present. The first one is to place soft clay slurry in a consolidation box and subsequently pre-consolidate the soft clay slurry on a consolidator. The second one is to use vacuum pre-consolidation, which can accelerate the percolation of pore water in soft clay. After the consolidation is completed, soil samples are obtained from bulk samples in the consolidation box with a sampler to carry out soil tests.

At present, soil samples prepared by using the foregoing methods are usually bulk soil samples, and cannot be directly used for indoor soil tests. Therefore, it is necessary to take samples from bulk soil samples. When an existing sampler is pressed into the bulk soil samples, disturbance to the consolidated soil samples is large, and cutting and separation from surrounding soil body are also difficult.

Therefore, a technical problem to be resolved by the present invention is to overcome the problem in the prior art that when an existing sampler is pressed into bulk soil samples, disturbance to consolidated soil samples is large, and cutting and separation from surrounding soil body are also difficult, and provide an indoor soil sample preparation instrument.

### Embodiment 1

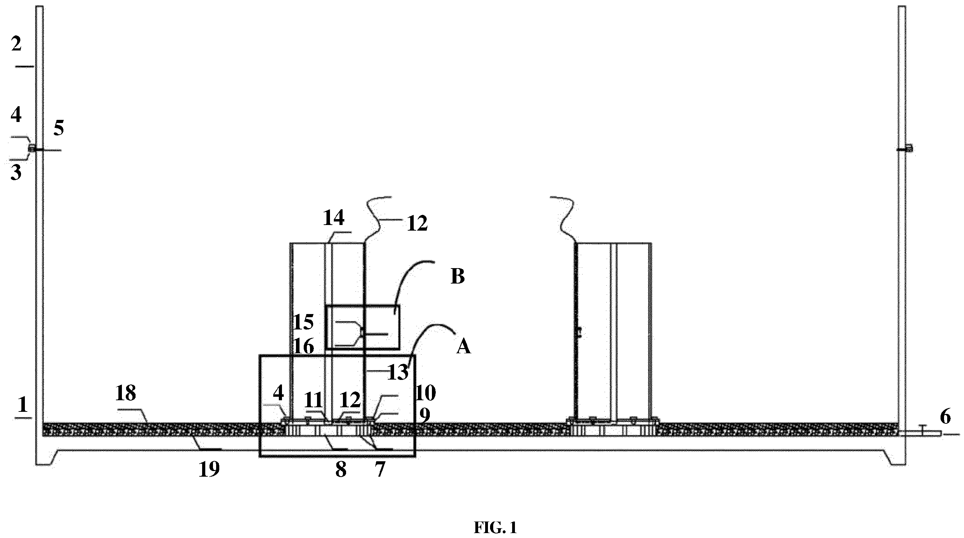

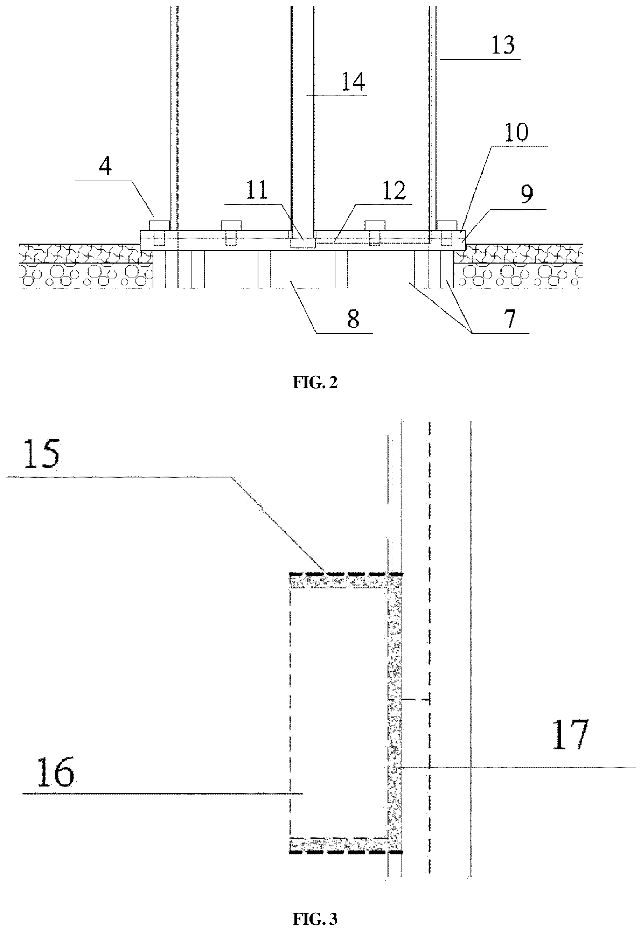

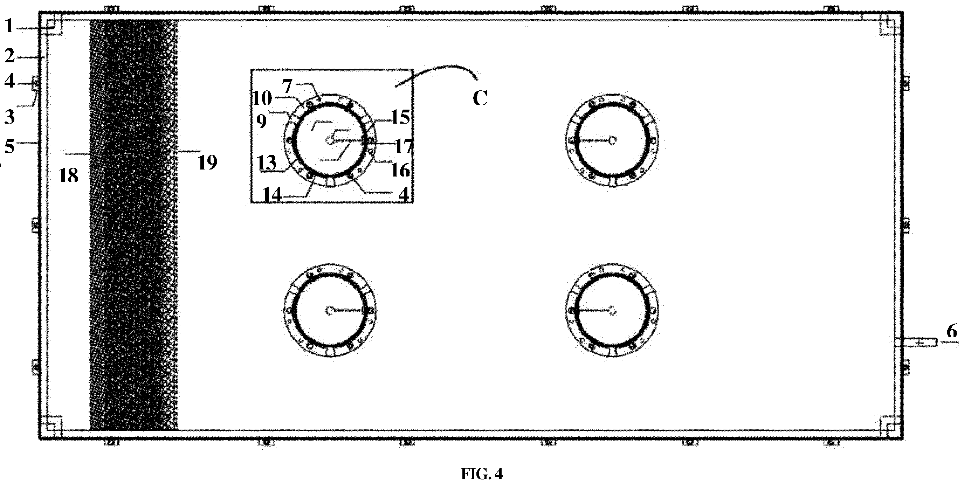

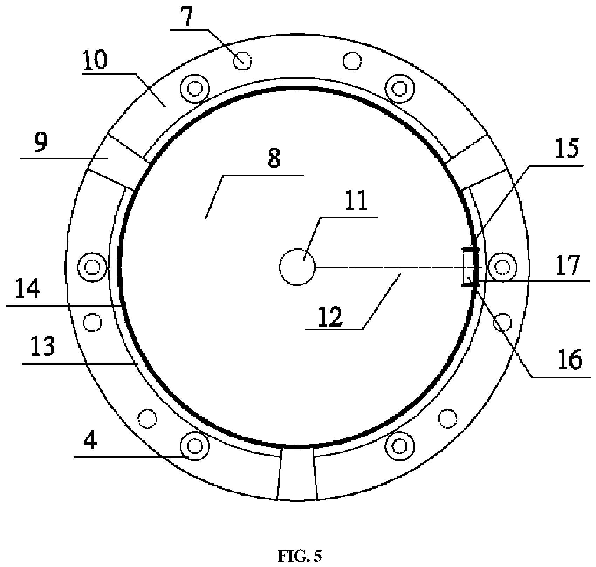

As shown in FIG. 1 to FIG. 5, the embodiments of the present invention provide an indoor soil sample preparation instrument. The indoor soil sample preparation instrument includes a consolidation box 1 and a sampling tube 14.

Specifically, a water filter layer is completely laid on a bottom of the consolidation box 1, and a drain is provided in the bottom of the consolidation box 1. Discharged water during the consolidation of a soil sample may seep through the water filter layer, and is then discharged through the drain. The sampling tube 14 has a tubular structure. The sampling tube 14 is disposed in the consolidation box 1. An end of the sampling tube 14 is connected to the water filter layer. A certain angle is formed between the sampling tube 14 and the water filter layer. That is, an end of the sampling tube 14 is directly inserted in the water filter layer. The sampling tube 14 may be vertically placed on the water filter layer or may be placed at 60°. Certainly, only an example of a placement angle of the sampling tube 14 is described in this embodiment, and does not constitute any limitation. A person skilled in the art may make a change according to an actual case, as long as the same technical effect can be achieved.

In such an arrangement, the sampling tube 14 is installed in the consolidation box 1 of the present invention. Compared with pressing of an existing sampler into a large soil sample, a conventional process of pressing a sampling tube 14 into soft clay is not required in the present invention, and sample preparation and sampling are integrated, so that disturbance to soil samples can be avoided, thereby improving sampling quality, and cutting and separation from surrounding soil body are easier.

Further, in an optional embodiment of the present invention, the consolidation box 1 has a rigid structure. Specifically, the consolidation box 1 may be made of stainless steel or aluminum alloy. In the embodiments of the present invention, a baffle 2 is disposed at a top of the consolidation box 1 in a circumferential direction. The baffle 2 and the consolidation box 1 may be detachably connected to each other. A person skilled in the art may arrange the baffle 2 to adjust the overall height and interior space of the consolidation box 1, so that a preparation amount of consolidated soil samples can be changed.

Further, in an optional embodiment of the present invention, the baffle 2 and the consolidation box 1 are correspondingly provided with a plurality of fixing sheets 3 at a connection location between the baffle 2 and the consolidation box 1, and a bolt hole for insertion of a bolt 4 is provided in each fixing sheet 3. In addition, a sealing strip 5 is laid at the connection location between the baffle 2 and the consolidation box 1. The sealing strip 5 may be a rubber strip or a silicone strip.

Certainly, only examples of the detachable connecting manner between the baffle 2 and the consolidation box 1 and the type of the sealing strip 5 are described in this embodiment, and does not constitute any limitation. A person skilled in the art may make a change according to an actual case, as long as the same technical effect can be achieved.

Further, in an optional embodiment of the present invention, a drain valve 6 is installed at the drain. In this way, A person skilled in the art may adjust the opening and closing of the drain valve 6 according to the status of remaining water in the consolidation box 1 during consolidation.

Further, in an optional embodiment of the present invention, a galvanization process is applied to a tube wall of the sampling tube 14. In the embodiments of the present invention, the sampling tube 14 can be kept from corrosion and the tube wall of the sampling tube 14 can be kept in a smooth state by applying the galvanization process to the tube wall of sampling tube 14, so that disturbance to soil samples can be avoided, thereby improving sampling quality, and cutting and separation from surrounding soil body are easier.

Certainly, only an example of an electroplating process of the sampling tube 14 is described in this embodiment, and does not constitute any limitation. A person skilled in the art may make a change according to an actual case, as long as the same technical effect can be achieved.

Further, in an optional embodiment of the present invention, the sampling tube 14 is a telescopic tube or is formed by connecting a plurality of tube rods. In the embodiments of the present invention, the baffle 2 is disposed to adjust the interior space of the instrument and the length of the sampling tube 14 is changed, so that sample preparation of soft clay of different scales can be implemented according to actual test requirements of soft clay in soil tests.

Further, the indoor soil sample preparation instrument further includes a plurality of arc-shaped vertical plates 13. The plurality of arc-shaped vertical plates 13 are disposed around a periphery of the sampling tube 14. The plurality of arc-shaped vertical plates 13 surround the sampling tube 14 in a space delimited by the plurality of arc-shaped vertical plates 13. Specifically, each arc-shaped vertical plate 13 has a rigid structure. In the embodiments of the present invention, the plurality of arc-shaped vertical plates 13 are disposed around the periphery of the sampling tube 14. The plurality of arc-shaped vertical plates 13 surround the sampling tube 14 in a space delimited by the plurality of arc-shaped vertical plates 13, so that the sampling tube 14 can be protected, to keep the sampling tube 14 from bending and damage.

Further, in an optional embodiment of the present invention, a slot hole 15 is opened in the arc-shaped vertical plate 13, and a hole pressure gauge 16 is installed in the slot hole 15. A sealing material 17 is filled between the hole pressure gauge 16 and the slot hole 15. The indoor soil sample preparation instrument further includes permeable stone 8. The permeable stone 8 is disposed at a bottom of the sampling tube 14. In addition, a soil pressure detection apparatus 11 is installed on the permeable stone 8. Specifically, a through hole is opened in the sampling tube 14 at a position corresponding to the slot hole 15. A transmission line 12 is provided in the through hole. The transmission line 12 needs to be protected by rubber. One end of the transmission line 12 is connected to the soil pressure detection apparatus 11. The other end of the transmission line 12 is connected to the hole pressure gauge 16. In the embodiments of the present invention, the soil pressure detection apparatus 11 and the hole pressure gauge 16 are installed on the sampling tube 14 and the permeable stone 8, so that the consolidation and preparation of soft clay can be accurately measured and controlled, and the consolidation status of soft clay can be acquired in real time. In the embodiments of the present invention, the soil pressure detection apparatus 11 may be a soil pressure sensor.

Further, in an optional embodiment of the present invention, a plurality of vertical posts are arranged around the permeable stone 8. A base ring 10 is installed upon the vertical posts 7. A plurality of flange boards 9 are disposed at intervals at bottoms of the arc-shaped vertical plates 13. The base ring 10 is connected to the flange boards 9. The flange boards 9 are used for fixing the arc-shaped vertical plates 13. The arc-shaped vertical plates 13 are used for fixing and protecting the sampling tube 14.

Further, in an optional embodiment of the present invention, the water filter layer includes a grit layer 19 and non-woven fabric 18. The grit layer 19 is laid at the bottom of the consolidation box 1. The grit layer 19 forms a consolidation drain path. The non-woven fabric 18 is laid flat on the grit layer 19.

Certainly, only an example of the specific structure of the water filter layer is described in this embodiment, and does not constitute any limitation. A person skilled in the art may make a change according to an actual case, as long as the same technical effect can be achieved.

A method for using the invention is as follows: (1) Determine a spatial size of the instrument. A volume of a soil sample to be prepared is determined according to requirements of an indoor soil test. Select and mount the sampling tube 14 and the baffle 2 to reach a corresponding height. (2) Pour and consolidate slurry: Pour well-stirred saturated slurry into the consolidation box 1, and after standing of the slurry, place the consolidation box 1 on a consolidometer to perform pre-consolidation, monitor a hole pressure of the soil pressure detection apparatus 11 at a bottom in a consolidation process, and accurately determine, according to data, whether consolidation is completed. (3) Perform sampling: after the consolidation is completed, remove the consolidation box 1 from the consolidometer, clean soft clay around the arc-shaped vertical plates 13 and the sampling tube 14, finally detach the arc-shaped vertical plates 13, extract the sampling tube 14, and store the sampling tube 14 in an indoor for future use.

Obviously, the foregoing embodiments are merely examples for clear description, rather than a limitation to implementations. For a person of ordinary skill in the art, other changes or variations in different forms may also be made based on the foregoing description. All implementations cannot and do not need to be exhaustively listed herein. Obvious changes or variations that are derived there from still fall within the protection scope of the invention of the present invention as defined in the appended claims.

## Claims

1. An indoor soil sample preparation instrument comprising:
a consolidation box (1), with a water filter layer being laid on a bottom thereof, and with a drain provided in the bottom of the consolidation box (1);
a sampling tube (14), having a tubular structure, the sampling tube (14) being disposed in the consolidation box (1), an end of the sampling tube (14) being connected to the water filter layer, a certain angle being formed between the sampling tube (14) and the water filter layer;
a baffle (2), disposed at a top of the consolidation box (1) in a circumferential direction;
a permeable stone (8), disposed at a bottom of the sampling tube (14), a soil pressure detection apparatus (11) being installed on the permeable stone (8); and
a plurality of arc-shaped vertical plates (13), disposed around a periphery of the sampling tube (14), the plurality of arc-shaped vertical plates (13) surrounding the sampling tube (14) in a space delimited by the plurality of arc-shaped vertical plates (13).

2. The indoor soil sample preparation instrument according to claim 1, wherein the consolidation box (1) has a rigid structure.

3. The indoor soil sample preparation instrument according to claim 2, wherein the consolidation box (1) is made of stainless steel or aluminum alloy.

4. The indoor soil sample preparation instrument according to any one of claims 1 to 3, wherein the baffle (2) and the consolidation box (1) are detachably connected to each other.

5. The indoor soil sample preparation instrument according to any one of claims 1 to 3, wherein the baffle (2) and the consolidation box (1) are correspondingly provided with a plurality of fixing sheets (3) at a connection location between the baffle (2) and the consolidation box (1), and a bolt hole for insertion of a bolt (4) is provided in each fixing sheet (3).

6. The indoor soil sample preparation instrument according to claim 5, wherein a sealing strip (5) is laid at the connection location between the baffle (2) and the consolidation box (1).

7. The indoor soil sample preparation instrument according to any one of claims 1 to 3, wherein a galvanization process is applied to a tube wall of the sampling tube (14).

8. The indoor soil sample preparation instrument according to any one of claims 1 to 3, wherein the sampling tube (14) is a telescopic tube or is formed by connecting a plurality of tube rods.

9. The indoor soil sample preparation instrument according to claim 8, wherein each arc-shaped vertical plate (13) has a rigid structure.

10. The indoor soil sample preparation instrument according to claim 9, wherein a slot hole (15) is opened in the arc-shaped vertical plate (13), and a hole pressure gauge (16) is installed in the slot hole (15).

11. The indoor soil sample preparation instrument according to claim 10, wherein a sealing material (17) is filled between the hole pressure gauge (16) and the slot hole (15).

12. The indoor soil sample preparation instrument according to claim 10 or 11, wherein a through hole is opened in the sampling tube (14) at a position corresponding to the slot hole (15), a transmission line (12) is provided in the through hole, one end of the transmission line (12) is connected to the soil pressure detection apparatus (11), and the other end of the transmission line (12) is connected to the hole pressure gauge (16).

13. The indoor soil sample preparation instrument according to claim 12, wherein a plurality of vertical posts are arranged around the permeable stone (8).

14. The indoor soil sample preparation instrument according to claim 13, wherein a base ring (10) is installed upon the vertical posts (7), a plurality of flange boards (9) are disposed at intervals at bottoms of the arc-shaped vertical plates (13), and the base ring (10) is connected to the flange boards (9).

15. The indoor soil sample preparation instrument according to claim 12, wherein the water filter layer comprises a grit layer (19) and non-woven fabric (18), the grit layer (19) is laid at the bottom of the consolidation box (1), the grit layer (19) forms a consolidation drain path, and the non-woven fabric (18) is laid flat on the grit layer (19).

## Patentansprüche

1. Instrument zur Vorbereitung von Bodenproben für den Innenbereich, umfassend:
einen Konsolidierungskasten (1), auf dessen Boden eine Wasserfilterschicht aufgebracht ist und der einen Ablauf im Boden des Konsolidierungskastens (1) aufweist;
ein Probenentnahmerohr (14), das eine röhrenförmige Struktur aufweist, wobei das Probenentnahmerohr (14) im Konsolidierungskasten (1) angeordnet ist, ein Ende des Probenentnahmerohrs (14) mit der Wasserfilterschicht verbunden ist und zwischen dem Probenentnahmerohr (14) und der Wasserfilterschicht ein bestimmter Winkel gebildet wird;
ein Ablenkblech (2), das an der Oberseite des Konsolidierungskastens (1) in einer Umfangsrichtung angeordnet ist;
einen durchlässigen Stein (8), der am Boden des Probenahmerohrs (14) angeordnet ist, wobei eine Bodendruck-Erfassungsvorrichtung (11) auf dem durchlässigen Stein (8) installiert ist; und
eine Vielzahl von bogenförmigen vertikalen Platten (13), die um einen Umfang des Probenentnahmerohrs (14) herum angeordnet sind, wobei die Vielzahl von bogenförmigen vertikalen Platten (13) das Probenentnahmerohr (14) in einem durch die Vielzahl von bogenförmigen vertikalen Platten (13) begrenzten Raum umgibt.

2. Instrument zur Vorbereitung von Bodenproben für den Innenbereich nach Anspruch 1, wobei der Konsolidierungskasten (1) eine starre Struktur aufweist.

3. Instrument zur Vorbereitung von Bodenproben für den Innenbereich nach Anspruch 2, wobei der Konsolidierungskasten (1) aus Edelstahl oder einer Aluminiumlegierung besteht.

4. Instrument zur Vorbereitung von Bodenproben für den Innenbereich nach einem der Ansprüche 1 bis 3, wobei das Ablenkblech (2) und der Konsolidierungskasten (1) lösbar miteinander verbunden sind.

5. Instrument zur Vorbereitung von Bodenproben für den Innenbereich nach einem der Ansprüche 1 bis 3, wobei das Ablenkblech (2) und der Konsolidierungskasten (1) an einer Verbindungsstelle zwischen dem Ablenkblech (2) und dem Konsolidierungskasten (1) entsprechend mit einer Vielzahl von Befestigungsblechen (3) versehen sind und in jedem Befestigungsblech (3) ein Bolzenloch zum Einsetzen eines Bolzens (4) vorgesehen ist.

6. Instrument zur Vorbereitung von Bodenproben für den Innenbereich nach Anspruch 5, **dadurch gekennzeichnet, dass** an der Verbindungsstelle zwischen dem Ablenkblech (2) und dem Konsolidierungskasten (1) ein Dichtungsstreifen (5) verlegt ist.

7. Instrument zur Vorbereitung von Bodenproben für den Innenbereich nach einem der Ansprüche 1 bis 3, wobei eine Rohrwand des Probenentnahmerohrs (14) galvanisiert wird.

8. Instrument zur Vorbereitung von Bodenproben für den Innenbereich nach einem der Ansprüche 1 bis 3, wobei das Probenentnahmerohr (14) ein Teleskoprohr ist oder durch Verbinden einer Vielzahl von Rohrstangen gebildet wird.

9. Instrument zur Vorbereitung von Bodenproben für den Innenbereich nach Anspruch 8, wobei jede bogenförmige vertikale Platte (13) eine starre Struktur aufweist.

10. Instrument zur Vorbereitung von Bodenproben für den Innenbereich nach Anspruch 9, wobei ein Langloch (15) in der bogenförmigen vertikalen Platte (13) ausgebildet ist und ein Lochdruckmesser (16) in dem Langloch (15) installiert ist.

11. Instrument zur Vorbereitung von Bodenproben für den Innenbereich nach Anspruch 10, wobei zwischen dem Lochdruckmesser (16) und dem Langloch (15) ein Dichtungsmaterial (17) eingefüllt ist.

12. Instrument zur Vorbereitung von Bodenproben für den Innenbereich nach Anspruch 10 oder 11, wobei ein Durchgangsloch in dem Probenentnahmerohr (14) an einer Position ausgebildet ist, die dem Langloch (15) entspricht, eine Übertragungsleitung (12) in dem Durchgangsloch vorgesehen ist, ein Ende der Übertragungsleitung (12) mit der Bodendruck-Erfassungsvorrichtung (11) verbunden ist und das andere Ende der Übertragungsleitung (12) mit dem Lochdruckmesser (16) verbunden ist.

13. Instrument zur Vorbereitung von Bodenproben für den Innenbereich nach Anspruch 12, wobei eine Vielzahl von vertikalen Pfosten um den durchlässigen Stein (8) herum angeordnet ist.

14. Instrument zur Vorbereitung von Bodenproben für den Innenbereich nach Anspruch 13, wobei ein Basisring (10) auf den vertikalen Pfosten (7) installiert ist, eine Vielzahl von Flanschplatten (9) in Abständen an den Böden der bogenförmigen vertikalen Platten (13) angeordnet sind und der Basisring (10) mit den Flanschplatten (9) verbunden ist.

15. Instrument zur Vorbereitung von Bodenproben für den Innenbereich nach Anspruch 12, wobei die Wasserfilterschicht eine Sandschicht (19) und einen Vliesstoff (18) umfasst, die Sandschicht (19) auf den Boden des Konsolidierungskastens (1) aufgebracht ist, die Sandschicht (19) einen Konsolidierungsabflussweg bildet und der Vliesstoff (18) flach auf die Sandschicht (19) gelegt ist.

## Revendications

1. Instrument de préparation d'échantillons de sol intérieur comprenant :
une boîte de consolidation (1), dont le fond est recouvert d'une couche de filtration d'eau, et dont le fond de la boîte de consolidation (1) est pourvu d'un drain ;
un tube d'échantillonnage (14) à structure tubulaire, le tube de prélèvement (14) étant placé dans la boîte de consolidation (1), une extrémité du tube d'échantillonnage (14) étant reliée à la couche de filtration de l'eau, un certain angle étant formé entre le tube d'échantillonnage (14) et la couche de filtration de l'eau ;
un déflecteur (2), disposé au sommet de la boîte de consolidation (1) dans le sens de la circonférence ;
une pierre perméable (8), disposée au fond du tube d'échantillonnage (14), un appareil de détection de la pression du sol (11) étant installé sur la pierre perméable (8) ; et
une pluralité de plaques verticales en forme d'arc (13), disposées autour d'une périphérie du tube d'échantillonnage (14), la pluralité de plaques verticales en forme d'arc (13) entourant le tube d'échantillonnage (14) dans un espace délimité par la pluralité de plaques verticales en forme d'arc (13).

2. Instrument de préparation d'échantillons de sol intérieur selon la revendication 1, dans lequel la boîte de consolidation (1) présente une structure rigide.

3. Instrument de préparation d'échantillons de sol intérieur selon la revendication 2, dans lequel la boîte de consolidation (1) est fabriquée en acier inoxydable ou en alliage d'aluminium.

4. Instrument de préparation d'échantillons de sol intérieur selon l'une quelconque des revendications 1 à 3, dans lequel le déflecteur (2) et la boîte de consolidation (1) sont reliés l'un à l'autre de manière amovible.

5. Instrument de préparation d'échantillons de sol intérieur selon l'une quelconque des revendications 1 à 3, dans lequel le déflecteur (2) et la boîte de consolidation (1) sont pourvus d'une pluralité de feuilles de fixation (3) à un emplacement de connexion entre le déflecteur (2) et la boîte de consolidation (1), et un trou de boulon pour l'insertion d'un boulon (4) est prévu dans chaque feuille de fixation (3).

6. Instrument de préparation d'échantillons de sol intérieur selon la revendication 5, dans lequel une bande d'étanchéité (5) est posée à l'emplacement de la connexion entre le déflecteur (2) et la boîte de consolidation (1).

7. Instrument de préparation d'échantillons de sol intérieur selon l'une quelconque des revendications 1 à 3, dans lequel un processus de galvanisation est appliqué à une paroi du tube d'échantillonnage (14).

8. Instrument de préparation d'échantillons de sol intérieur selon l'une quelconque des revendications 1 à 3, dans lequel le tube d'échantillonnage (14) est un tube télescopique ou est formé par la connexion de plusieurs tiges de tube.

9. Instrument de préparation d'échantillons de sol intérieur selon la revendication 8, dans lequel la plaque verticale en forme d'arc (13) présente une structure rigide.

10. Instrument de préparation d'échantillons de sol intérieur selon la revendication 9, dans lequel une fente (15) est ouverte dans la plaque verticale en forme d'arc (13), et un manomètre (16) est installé dans la fente (15).

11. instrument de préparation d'échantillons de sol intérieur selon la revendication 10, dans lequel un matériau d'étanchéité (17) est versé entre le manomètre du trou (16) et le trou de la fente (15).

12. Instrument de préparation d'échantillons de sol intérieur selon la revendication 10 ou 11, dans lequel un orifice passant est ouvert dans le tube d'échantillonnage (14) à une position correspondant au trou de fente (15), une ligne de transmission (12) est ménagée dans l'orifice passant, une extrémité de la ligne de transmission (12) est raccordée à l'appareil de détection de la pression du sol (11), et l'autre extrémité de la ligne de transmission (12) est raccordée au manomètre du trou (16).

13. Instrument de préparation d'échantillons de sol intérieur selon la revendication 12, dans lequel plusieurs poteaux verticaux sont disposés autour de la pierre perméable (8).

14. Instrument de préparation d'échantillons de sol intérieur selon la revendication 13, dans lequel un anneau de base (10) est installé sur les montants verticaux (7), une pluralité de planches à brides (9) sont disposées à intervalles au bas des plaques verticales en forme d'arc (13), et l'anneau de base (10) est relié aux planches à brides (9).

15. Instrument de préparation d'échantillons de sol intérieur selon la revendication 12, dans lequel la couche de filtration d'eau comprend une couche de gravier (19) et un tissu non tissé (18), la couche de gravier (19) est placée au fond de la boîte de consolidation (1), la couche de gravier (19) forme un chemin de drainage de consolidation, et le tissu non tissé (18) est disposé à plat sur la couche de gravier (19).
